(19) Europäisches Patentamt
European Patent Office
Office européen des brevets

(11) **EP 2 785 354 B1**

(12) **EUROPEAN PATENT SPECIFICATION**

(45) Date of publication and mention
of the grant of the patent:
**09.09.2020 Bulletin 2020/37**

(21) Application number: **12791511.4**

(22) Date of filing: **29.11.2012**

(51) Int Cl.:
*A61K 31/7048* (2006.01)   *A61K 36/535* (2006.01)
*A61K 36/00* (2006.01)   *A61P 25/16* (2006.01)
*A61P 25/28* (2006.01)   *A61P 25/24* (2006.01)
*A61P 25/22* (2006.01)   *A61P 25/20* (2006.01)
*A61P 25/14* (2006.01)   *A61K 45/06* (2006.01)

(86) International application number:
**PCT/EP2012/074010**

(87) International publication number:
**WO 2013/079624 (06.06.2013 Gazette 2013/23)**

(54) **COMPOSITION COMPRISING VICENIN-2 HAVING A BENEFICIAL EFFECT ON NEUROLOGICAL AND/OR COGNITIVE FUNCTION**

ZUBEREITUNG ENTHALTEND VICENIN-2 MIT VORTEILHAFTER WIRKUNG BEI NEUROLOGISCHEN UND COGNITIVEN FUNKTIONEN

FORMULATION COMPRENANT DU VICENIN-2 PRÉSENTANT DES EFFETS BENEFIQUES POUR LES FONCTIONS NEUROLOGIQUES ET COGNITIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 EP 11009430**
**29.11.2011 US 201161564374 P**
**10.04.2012 EP 12163578**
**10.04.2012 US 201261622260 P**

(43) Date of publication of application:
**08.10.2014 Bulletin 2014/41**

(73) Proprietor: **Amino Up Chemical Co., Ltd.**
**Sapporo-shi, Hokkaido 004-0839 (JP)**

(72) Inventors:
• **BUCHWALD-WERNER, Sybille**
**40589 Düsseldorf (DE)**
• **FUJII, Hajime**
**Ebetsu, Hokkaido 069-0853 (JP)**

(74) Representative: **Witthoff Jaekel Steinecke Patentanwälte PartG mbB**
**Postfach 1140**
**52412 Jülich (DE)**

(56) References cited:
• **L. M. SENA ET AL: "Neuropharmacological Activity of the Pericarp of Passiflora edulis flavicarpa Degener: Putative Involvement of C-Glycosylflavonoids", EXPERIMENTAL BIOLOGY AND MEDICINE, vol. 234, no. 8, 1 August 2009 (2009-08-01), pages 967-975, XP055026536, ISSN: 1535-3702, DOI: 10.3181/0902-RM-84**

**Description**

**Technical field**

The invention relates to a food, a dietary supplement and a drug composition comprising as an active ingredient vicenin 2, and especially its beneficial effects to maintain and/or improve neurological and brain function and to prevent, delay onset, control and/or treat a cognitive dysfunction, condition, disorder or disease, e.g. dementia such as Alzheimer disease.

**Background of the invention**

Maintenance or improvement of neurological and brain functions is important to vitality and well being throughout all stages of life but particularly important for elderly people. The efficiency of each person's neurological and brain functions can make all the difference in daily and overall health. The ability of cognitive functions, including memory, attention, concentration, alertness, mental flexibility and speed, learning, intelligence, language, problem solving capacity, consciousness, coping with psychological stress or tension, motivation, mobility, decision making and reaction time as well as emotions like anxiety and mood swings affect social and economical status and the overall quality of life. The wish to extend cognitive capacity longer, frame a lifelong challenge, as everybody is effected by aging. In addition, there is an increasing number of individuals, who develop neurodegenerative diseases, like dementia or Alzheimer disease. [1, 2]

Dementia can be divided into three types, referring to their cause of development. The first type is called vascular dementia, caused by reduced circulation; the second type is called secondary dementia, generated as a side effect of e.g. hormonal disorders and the third one, is Alzheimer. Alzheimer disease is the most common form of dementia, forming up to 60% of the cases. There is no cure for the disease, which worsens as it progresses, and eventually leads to death.

Alzheimer is a devastating neurological disorder that affects more than 37 million people worldwide. The economic burden of Alzheimer's disease is massive and currently approved drugs do not prevent or reverse the disease and provide only modest symptomatic benefits. [3] The pathogenesis of Alzheimer disease is not completely understood. Major characteristics of Alzheimer's disease (AD) are synaptic loss, cholinergic dysfunction, and abnormal protein depositions in the brain. A combination of several causes may lead to the development of plaques in the brain, which decrease the efficiency of neuronal communication leading to death of neurons. Scientifically these plaques are called "beta amyloid peptides" or "Tau-proteins". [3, 4] Recent studies confirmed that a genetic disposition may be involved and/or infection may contribute to the formation of plaques and the development of Alzheimer disease. The decline in neurological and brain functions is linked to the reduced number of neurons, which lead to a reduced synthesis of the neurotransmitter acetylcholine and glutamine. The increase of neurotransmitter concentration, by inhibiting its metabolism is a possibility to promote beneficially the neurological and brain function or to reduce the development of dementia like Alzheimer disease. [4]

Not many drugs are approved for the treatment of dementia such as Alzheimer. Four approved drugs target the increase of neurotransmitter concentration. Recent drug developments and areas of clinical research focus on treating the cause of the disease by reduction of amyloid beta levels. Immunotherapy or vaccinations with anti-amyloid antibodies are being investigated. Another substance, called PBT2 binds metals which are necessary for building of protein structures forming the plaques. Other proteins may have the ability to cut amyloid beta into small pieces, which can be eliminated. In addition, gab junction antagonists are being investigated. They block confused communication between cells to promote a healthy neurological and brain functionality. [3, 4] As of 2012, more than 1000 clinical trials have been or are being conducted to find ways to treat Alzheimer disease. [11] Sena et al. (2009) Experimental Biology and Medicine, 234:967-975 describes a phytochemical investigation of extracts from Passiflora edulis showing C-glycosyl flavonoids i.a. vicenin-2, in aqueous extract and butanolic fraction of Passiflora edulis. Despite considerable efforts by academic researchers and pharmaceutical and food industry, the development of novel ingredients for the maintenance and improvement of neurological and brain function and to prevent the development and to slow down the progression of dementia and Alzheimer disease has been dragging and did not lead to many innovations. Thus, there is a need to develop drugs, functional foods and dietary supplement ingredients which may contribute to the maintenance and improvement of neurological and brain function and which may prevent the development of cognitive disorders or slow down the progression of dementia and Alzheimer disease.

The object for the present invention was to provide novel active ingredients suitable as functional food, dietary supplement ingredients and drugs, which are able to maintain and improve neurological and brain function and to prevent, delay onset, control and treat cognitive dysfunctions, conditions, disorders or diseases, in particular dementia such as Alzheimer disease.

## Summary of the invention

In accordance with the present invention it has been surprisingly found that the active ingredient vicenin 2 and compositions comprising the same have beneficial effects on cognitive functions including memory, attention, concentration, alertness, mental flexibility and/or speed, learning, intelligence, language, problem solving capacity, consciousness, coping with psychological stress or tension, motivation, mobility, decision making capacity and reaction time as well as emotions like anxiety and mood swings.

Thus, the invention describes a new neurological and cognitive active agent which helps to maintain and/or improve neurological and brain function and/or to prevent and/or improve cognitive changes and to prevent and/or treat cognitive disorders like dementia. Preferably, dementia is Alzheimer disease. The cognitive changes may be stress or age related cognitive changes. These changes may be mild.

In particular, the invention relates to vicenin 2 as an active ingredient for use in preventing, delaying onset, controlling and/or treating a neurological dysfunction, condition, disorder or disease, wherein the neurological dysfunction, condition, disorder or disease is an age or stress related neurological dysfunction, a cognitive disorder, depression or dementia, wherein dementia is preferably Alzheimer disease.

Furthermore, the invention further relates to a non-therapeutic use of vicenin 2 as an active ingredient for maintaining and/or improving neurological and/or brain function, wherein the neurological and/or brain function is selected from the group consisting of memory, attention, concentration, alertness, mental flexibility and/or speed, learning, intelligence, language skills, problem solving capacity, consciousness, coping with psychological stress or tension, motivation, mobility, decision making capacity, reaction time and regulation of emotions.

Preferably, the neurological condition is associated with impaired neurological and/or brain function. In other words, preferably, the maintaining and/or improving neurological and/or brain function may be associated with a neurological dysfunction, condition, disorder or disease.

Thus, the present invention may relate to vicenin 2 as an active ingredient for use in preventing, delaying onset, controlling and/or treating a neurological dysfunction, disorder, disease or condition, which is associated with impaired neurological and/or brain function.

In a further embodiment, the invention relates to a use of vicenin 2 as an active ingredient for maintaining and/or improving neurological and/or brain function. This embodiment relates to a use of vicenin 2 for healthy individuals, who wish to e.g. enhance cognitive performance and/or improve well-being at stress situations.

In a further embodiment, the invention relates to vicenin 2 as an active ingredient for use in improving cognitive functions, memory, attention, concentration, alertness, mental flexibility and/or speed, learning, intelligence, language, problem solving capacity, consciousness, coping with psychological stress or tension, motivation, mobility, decision making capacity, reaction time, and/or mood swings in a patient suffering from a neurological dysfunction, disorder or disease.

Preferably, the neurological dysfunction, condition, disorder or disease is an age or stress related neurological dysfunction, a cognitive disorder, depression or dementia, wherein dementia is preferably Alzheimer disease. Furthermore, the active ingredient is preferably derived from a plant and the active ingredient is a plant preparation enriched for the active ingredient. Preferably, the plant is selected from a group consisting of a Anethum, Perilla, Urtica, Passiflora, Camelia, Cayaponia, Colocasia, Cydonia, Desmodium, Hordeum, Origanum, Ocimum, Jatropha, Parkinsonia, Peperomia, Piheranthos, Centaurea, Indigo, Bomba, Lychnophera, Asplenium, Chinotto, Citrus, Viola, Trigonella, Rosemary, Peppermint, Thyme, Basil, Sage, Oregano, Lavandula, Nipponanthemum, Abrus, Viola, Santalum, Oryza, Scleropyrum, Tulsi, Centaurea, Indigofera, Bombax, Glinus, Lychnophora and other species belonging to the Lamiacea, Labiatae, and Urticaceae, Rosales or Malpighiales or a combination of said plants.

The plant preparation may be selected from the group consisting of a leaf preparation, a fruit preparation, a seed preparation, a stem preparation, a flower preparation, a bud preparation, a root preparation or a mixture of different parts of the plant.

In one embodiment, the active ingredient is an isolated vicenin 2 thereof obtained by isolation or chemical synthesis.

In a preferred aspect of the present invention, the neurological and/or brain function is selected from the group consisting of memory, attention, concentration, alertness, mental flexibility and/or speed, learning, intelligence, language skills, problem solving capacity, consciousness, coping with psychological stress or tension, motivation, mobility, decision making capacity, reaction time and regulation of emotions. The emotions may be e.g. mood swings. These functions may be impaired in association with a cognitive disorder such as neurodegenerative disorder or dementia.

The active ingredient may be comprised in a preferred embodiment in a composition such as a food product, dietary supplement or medicament, preferably wherein the medicament comprises a pharmaceutically acceptable carrier. In these aspects, the concentration of the active ingredient is from 0.1 μg to 500 μg, preferably from 2.5 μg to 50 μg, preferably from 5 μg to 15μg or 12 μg to 30 μg, most preferably about 24 μg.

Most preferably, the active ingredient is administered at a dose of 0.1 - 0.5 μg/kg, 0.17 - 0.42μg/kg or 0.07 -

0.3 μg/kg.

Furthermore, in a preferred embodiment the active ingredient may be comprised in globules, pellets, powder formulations, tablets, capsules, stick formulations, sachet formulations or a fluid. The fluid may be in a bottle with a dropper.

Preferably, the composition is substantially free of other plant flavonoids derived from the same plant or other plant extracts or free of flavonoids of other plants.

In another preferred embodiment, the composition comprises one or more further agents capable of maintaining and/or improving neurological and/or brain function and/or preventing, delaying onset, controlling and/or treating a neurological dysfunction, condition, disorder or disease as defined in any one of the preceding claims. This agent may be a lipid, a lipid containing omega-3-fatty acid, a physiologically active fatty acid, an antioxidant, an anti-inflammatory agent, a bulking agent, an immune system modulatory agent or a vaccine, an antibody, a metal-protein interaction attenuation agent, a plant preparation, curcumin, coenzym Q10, L-carnitine, zinc, epigallocatechingallate, thymol, p-cymere, vinpocetine, hyperzine A, phosphatidylserine, a vitamin, alpha liponic acid, a TNF alpha inhibitor, a flavonoid, an anthocyanidin, a biflavonoid, a flavon, a flavonglycoside or a carboxylic acid.

Preferably, the plant preparation is selected from one or more extracts from a group consisting of an extract of Ginkgo biloba, Hypericum perforatum, Hyperzia serrata, Galanthus nivalis, Salvia officinalis, Panex ginseng, Lippia citriodora, Melissa officinalis, Passiflora incarnate, Passiflora edulis, Bacopa monniera, Zingiber officinalis, Leucojum aestrum, Concolulus pluricaulis and Centella asiatica, Emblica officinalis, Coptidis Rhizoma, Salvia triloba, Piper nigrum, Trigonella foenum-graecum, Cimicifuga racemosa, Salvia miltiorrhiza, Rhodiola rosea, Habranthus jamesonii, Phycella herbertiana, Rhodophiala mendocina, Zephyranthes filifolia, Stephania pierrei, Kaempfera parviflora, Stephania venosa, Crocus sativus, Salvia species, Bacopa monnieri, Centella asiatica, Ptychopetalum olacoides, Withania somnifera, Coptis chinensis, Mangifera indica, Polygala caudata, Polygala tenuifolia, Halenia elliptica, Evolvilus alsinoides, Celastrus paniculatus, Clitoria ternatea, Curcuma longa, Acorus calamus, Terminalia chebula, Lycoris radiata, Magnolia officinalis, Biota orientalis, Codonopsis pilosula, Evodia rutaecarpa, Polygonum multiflorum, Aspalathus linearis, Cyclopia species, Adansonia digitata, Sclerocarya birrea, Actinidia chinensis, Matricaria recutita or combinations thereof. In a more preferred embodiment, the plant preparation is a preparation of Melissa officinalis, Rhodiole rosea, Magnifera indica or a Cyclopie species or any combination thereof.

In a preferred embodiment, the agent capable of maintaining and/or improving neurological and/or brain function and/or preventing, delaying onset, controlling and/or treating a neurological dysfunction, condition, disorder or disease is an acetylcholinesterase inhibitor, a NMDA (N-methyl-D-aspartate) receptor inhibitor, a non steroidal anti-rheumatic agent, a neuroleptic agent, a tricyclic antidepressant, an anti-psychotic agent, a gab junction inhibitor, a non selective monoaminooxidase inhibitor, an ABCC1 Transporter, an ADAM 10 protein, methylthioninium chloride, an antibiotic agent, an antiviral agent, a gamma secretase inhibitor, a beta secretase inhibitor, an angiotensin receptor antagonist (AT1 antagonist), a cannabinoid, allopregnanolone or an insulin sensitizer.

As an example the active ingredient may be obtained by a process of producing a preparation of the active ingredient as defined above comprising the steps of:

a) selection of the raw plant material,

b) preparation of the raw plant material,

c) applying an extraction process using solvent extraction and/or filtration techniques, preferably followed by concentration and/or spray drying of the liquid extract into a powder,

d) determining the concentration of the active ingredient; and

e) selecting the preparation comprising the active ingredient at a concentration of at least 0.01%.

The process may comprise an extraction process using aqueous solvent extraction, more preferably water extraction.

The solvent may be water. The extraction may be carried out at a temperature of 90°C to 125°C and/or the temperature of the solvent is 55°C or above, preferably 70°C or above.

Preferably, the plant is selected from one or more plants of the group consisting of Anethum, Perilla, Urtica, Passiflora, Camelia, Cayaponia, Colocasia, Cydonia, Desmodium, Hordeum, Origanum, Ocimum, Jatropha, Parkinsonia, Peperomia, Piheranthos, Centaurea, Indigo, Bomba, Lychnophera, Asplenium, Chinotto, Citrus, Viola, Trigonella, Rosemary, Peppermint, Thyme, Basil, Sage, Oregano, Lavandula, Nipponanthemum, Abrus, Viola, Santalum, Oryza, Scleropyrum, Tulsi, Centaurea, Indigofera, Bombax, Glinus, Lychnophora and other species belonging to the Lamiacea, Labiatae, Urticaceae, Rosales or Malpighiales or combinations thereof.

Preferably, the preparation comprises the active ingredient at a concentration of at least, 0.02%, more preferably, at a concentration of at least 0.1 %, 0.15%, 0.2% or most preferably at a concentration of at least 0.2%, 0.25% or 0.3 %.

In some cases, the preparation may comprise the active ingredient at a concentration of at least 0.001%, more preferably, at a concentration of at least 0.002%, 0.005%, 0.008% or preferably at a concentration of at least 0.010%, 0.015% or 0.02%.

In some cases, the preparation may comprise the active ingredient at a concentration of at least 0.2%, more preferably, at a concentration of at least 0.5%, 1.5%, 3.0% or most preferably at a concentration of at least 2.0%, 3.0% or 5.0%.

Detailed descriptions of conventional extraction methods, such as those employed herein can be found in the literature, for example in the book "Industrial Scale Natural Products Extraction", published in 2011 by Wiley-VCH.

The analytical method to determine the vicenin 2 concentration may be e.g. a chromatographic method called high performance liquid chromatography. High performance liquid chromatography (HPLC) is one of the most popular techniques of analytical chemistry. A UV detector is used, detecting at 320nm. For determining the concentration of vicenin 2, a vicenin 2 reference or its derivates may be used as reference material. For example, apigenin may be used as reference substance and the vicenin 2 content is calculated via the difference in molecule weight and the slope and intercept of the calibration curve for apigenin.

In the process, the raw plant material may be Perilla species, more preferably Perilla frutescens Britton var.crispa or var.acuta Kudo. The raw plant material is preferably prepared by drying, cutting and/or milling. Extraction can be preferably done with a raw material which particle size is reduced to lower than 2mm$^2$. The solvent may be, preferably, water, methanol, ethanol, propanol, isopropanol, ethyl acetate, hexane, chloroform or dichloromethane. An aqueous solvent, e.g. water, may be used since glycosides, like vicenin 2, which have hygroscopic properties, are extracted by hot water more efficiently than alcohol. Moreover, some volatile, allergenic compounds like Perillaldehyde, Methyleugenol and Myristicin, which may occur in targeted species containing vicenin 2, can be eliminated by hot water.

Ratio of raw material to solvent may be between 1:100 to 20:100 and more preferably 2:100 to 10:100.

Extraction may be done by room temperature to up to 150°C. Extraction is more preferably carried out from 90°C to 125°C. In a further preferred embodiment, heat with additional pressure can be used.

The extraction time may be 10 min to 2 hours, more preferably from 20 min to 50 min.

The plant for the process may be selected from one or more plants from the group consisting of Anethum, Perilla, Urtica, Passiflora, Camelia, Cayaponia, Colocasia, Cydonia, Desmodium, Hordeum, Origanum, Ocimum, Jatropha, Parkinsonia, Peperomia, Piheranthos, Centaurea, Indigo, Bomba, Lychnophera, Asplenium, Chinotto, Citrus, Viola, Trigonella, Rosemary, Peppermint, Thyme, Basil, Sage, Oregano, Lavandula, Nipponanthemum, Abrus,Viola, Santalum, Oryza, Scleropyrum, Tulsi, Centaurea, Indigofera, Bombax, Glinus, Lychnophora and other species belonging to Lamiaceae, Labiatae, Urticaceae, Rosales or Malpighiales or combinations thereof.

This process provides an extract with a higher concentration of the active ingredient as the prior art processes due to concentration of the active ingredient. The higher concentration is achieved using state of the art extraction equipment, which allows an optimal interaction between the raw material and the extraction solvents. All critical process steps, for example temperature, are controlled in process at any times and adaptations are continuously possible to ensure highest yield for the active ingredient. Due to the selection of the extraction solvents combined with optimized physical conditions it is possible to diminish unwanted substances which may occur naturally in the different raw materials.

In addition, the present invention provides a kit comprising an active ingredient as defined above, the composition as defined above or the preparation as defined above and instructions for administering said composition. Preferably, the kit is for use in maintaining and/or improving neurological and/or brain function and/or preventing, delaying onset, controlling and/or treating a neurological dysfunction, condition, disorder or disease.

The present invention further provides a composition comprising the active ingredient as defined above, the preparation as defined above or the composition as defined above for use in preventing, delaying onset, controlling and/or treating a disorder or disease associated with autonomous neuronal death by blocking the gap junction hemichannel of activated microglia and reducing their glutamate release. Preferably, said disorder or disease is a neurodegenerative disorder, such as Alzheimer disease, Parkinson disease, Huntington disease, multiple sclerosis, amylotrophic lateral sclerosis (ALS), viral encephalitis or AIDS. Preferably, said composition comprises or is derived from a plant preparation comprising the active ingredient. More preferably, the plant preparation is derived from a plant and/or plant preparation as defined above. In this embodiment the composition is preferably Perilla extract.

In a further embodiment, the present invention relates to a composition comprising the active ingredient, i.e. vicenin 2 or biologically active analogue as thereof, as defined above or the preparation as defined above, or the composition as defined above for use in preventing, delaying onset, controlling and/or treating a condition associated with glutamate and tumor necrosis factor $\alpha$ released by activated microglia inducing excitotoxic neuronal death. In this embodiment, the composition is preferably Perilla extract.

Furthermore, the present invention relates to a composition comprising the active ingredient having one or more effects, preferably more than one, more preferably more than two, more than three, more than four or more than five effects, selected from the group consisting of reversible acetylcholinesterase inhibition, blockade of gap junction hemichannels, neuroleptic effect, anti-depressive effect, enkephalin like effect and reduction in TNF alpha for use in maintaining and/or improving neurological and/or brain function and/or preventing, delaying onset, controlling and/or treating neurological dysfunction, condition, disorder or disease. Preferably, said condition is associated with impaired neurological and/or brain function.

In a further embodiment, the invention relates to a vicenin 2 or a functionally active derivative thereof as an active ingredient for use in maintaining and/or improving neurological and/or brain function and/or preventing, delaying onset, controlling and/or treating a neurological dysfunction, condition, disorder or disease, wherein the active ingredient is not derived from Perilla.

**Brief description of the Figures:**

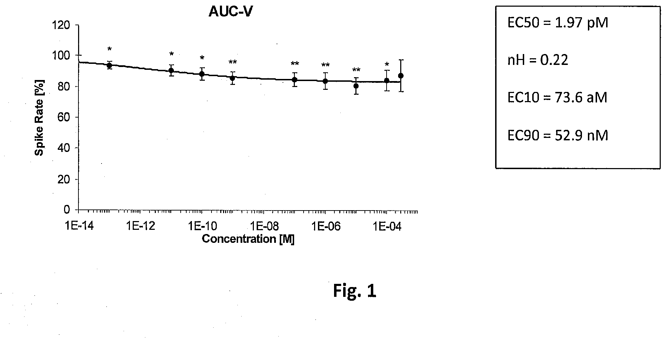

**Figure 1:** Acute effects of vicenin 2 (AUC-V) on the cortical network activity in vitro. Plotted are the spike rate changes for treatment of 9 accumulating concentrations in the range of 10 pg/ml to 300 $\mu$l/ml (mean $\pm$ standard error, n=17; Student's paired t-test: * $p \leq 0.05$; ** $p \leq 0.01$; ***$p \leq 0.001$).

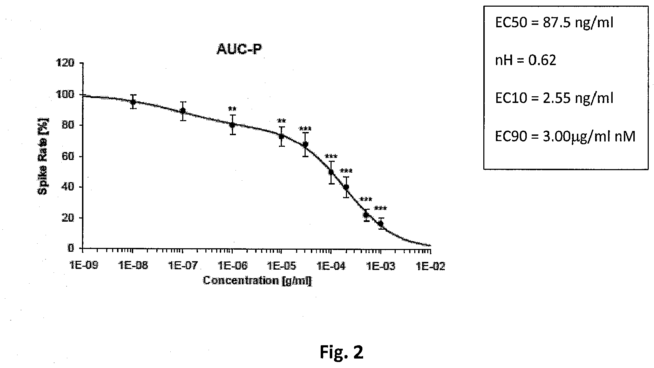

**Figure 2:** Acute effects of Perilla leaf extract (AUC-P) on the cortical network activity in vitro. Plotted are the spike rate changes for treatment of 9 accumulating concentrations in the range of 10 ng/ml to 1mg/ml (mean $\pm$ standard error, n=15; Student's paired t-test: *$p \leq 0.05$; ***$p \leq 0.01$; ***$p \leq 0.001$).

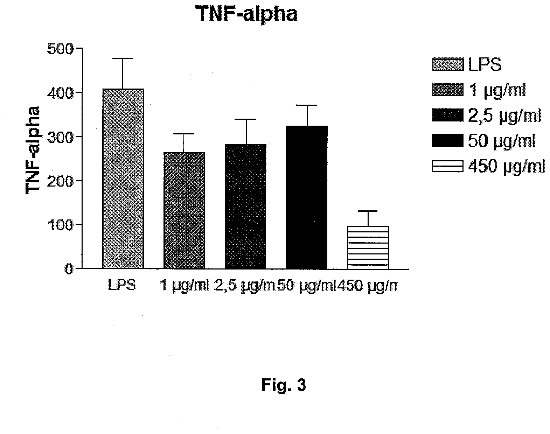

**Figure 3:** A dose-depending effect of a plant extract comprising vicenin 2 was shown to reduce TNF$\alpha$. TNF alpha secretion [pg/ml] after co- incubation with LPS and Perilla extract of different concentration (1$\mu$g/ml, 2.5 $\mu$g/ml and 50$\mu$g/ml) against 450 $\mu$g/ml a benchmark blend, containing e.g. Boswellia extract, Omega 3 fatty acids, ALA and Curcumin.

**Detailed description of the invention**

The inventors surprisingly found an agent having beneficial effects on neurological function and brain health, like cognitive functions, including memory, attention, concentration, alertness, mental flexibility and/or speed, learning, intelligence, language, problem solving capacity, consciousness, coping with psychological stress or tension, motivation, mobility, decision making capacity and reaction time as well as emotions like anxiety and mood swings.

In particular, the inventors surprisingly found that vicenin 2 thereof have beneficial effects to maintain and improve neurological and brain function and to prevent and improve age or stress related cognitive changes, which may be mild, and to prevent and treat cognitive disorders, for example dementia, in particular Alzheimer disease. This efficacy is very beneficial for cognitive functions, including memory, attention, concentration, alertness, mental flexibility and/or speed, learning, intelligence, language skills, problem solving capacity, consciousness, coping with psychological stress or tension, motivation, mobility, decision making and reaction time as well as emotions like anxiety and mood swings.

Although Alzheimer disease develops differently for every individual, there are many common symptoms. Early symptoms are decrease in memory, concentration and alertness, escalating into confusion, mood swings and emotional difficulties, loss in orientation, language problems and loss of long-term memory. Even early symptoms lead to a disconnection to family and society, which triggers mood swings, including aggression as well as anxiety. In the advanced status of Alzheimer disease body functions decrease ultimately leading to death. [4]

The pathogenesis of Alzheimer disease is not completely understood and a combination of several causes may lead to the development of plaques in the brain, which decrease the efficiency of neuronal communication leading to die off of neurons. Scientifically these plaques are called "beta amyloid peptides" or " Tau-proteines". Recent studies confirmed that a genetic disposition may be involved and/or infection may contribute to the formation of plaques and the development of Alzheimer disease. [3, 4]

First-line therapy for all forms of dementia, including Alzheimer, is intellectual activities, such as reading, playing board games, completing crossword puzzles, playing musical instruments, or regular social interaction. Life experiences result in more efficient neural functioning providing the individual a cognitive reserve that delays the onset of dementia manifestations. Physical activity may also be associated with a reduced risk of dementia and the slowdown in progression. Psychosocial therapies to reduce daily stress and anxiety are as important as dietary modifications to increase the intake of antioxidants. [4]

Several dietary supplement products are available to support brain health. Most of them are linked to antioxidant and anti-aging efficacy, for example, long chain omega 3 fatty acids, an physiological active fatty acid, eicosapenteanoic acid EPA, docosahexeanoic acid DHA, Ginkgo biloba extract, Curcumin or other polyphenols, catechins, flavonoids or phenolic carboxylic acid based plant preparations, including TCMs. Choline and Uridine monophosphate are also used as well as Vitamins, like folic acid and the pyridoxine (B6) and B12, vitamin C, vitamin E and selenium. The functional food products for these areas can also be defined as medical food which use and intake is to be recommended and supervised by a medical doctor. The medical food market segment is a developing market segment.

The invention provides a new important possibility of application for brain health, to maintain and improve neurological and brain function and to prevent and improve age or stress related associated cognitive changes, which may be even mild, and to prevent and treat cognitive disorders, dementia and Alzheimer disease.

Surprisingly, as shown by the Examples the active ingredient of the invention and the composition comprising the active ingredient demonstrate a plurality of modes of action beneficial for neurological and/or brain function. Thus, the composition comprising the active ingredient can be used for treating an individual suffering from several conditions. These may include conditions associated with reversible acetylcholinesterase inhibition, blockade of gap junction hemichannels and reduction in TNF alpha. Furthermore, the active ingredient as well as the composition comprising the same show neuroleptic effect and anti-depressive effects. Therefore, the active ingredient and compositions comprising the same result in a higher success rate in the treatment of an individual suffering from a neurological dysfunction, condition, disorder or disease. The disorder may be a neurodegenerative disorder, preferably Alzheimer disease, Parkinson disease, Huntington disease, multiple sclerosis, amylotrophic lateral sclerosis (ALS), encephalitis or AIDS.

In addition to above, the active ingredient may be used in treatment of individuals suffering from more than one dysfunctions, conditions, diseases or disorders. These may include e.g. dementia, in particular Alzheimer disease, a disorder associated to neuronal cell death or inflammation.

A further advantage of the composition of the invention comprising the active ingredient is that it has a plurality of therapeutic properties; i.e. the active ingredient or composition comprising the same has one or more of the following properties (i) an anti-inflammatory effect, (ii) anti-nociceptic effect, (iii) sedative effect, (iv) anxiolytic effect, (v) anti-cancer effect, (vi) immuno-modulation inducing effect and/or (vii) a beneficial effect on cognitive behavior and/or mood.

## Definitions

It is to be noted that the term "a" or "an" entity refers to one or more of that entity; for example, "an antibody", is understood to represent one or more antibodies. As such, the terms "a" (or "an"), "one or more", and "at least one" can be used interchangeably herein.

By an "isolated" ingredient, variant, or derivative thereof is intended an agent that is not in its natural milieu. No particular level of purification is required. For example, an isolated active ingredient can be removed from its native or natural environment. Synthetically produced active ingredients are considered "isolated" for purpose of the invention, as are native or synthetic active ingredients which have been separated, fractionated, or partially or substantially purified by any suitable technique.

By "subject" or "individual" or "animal" or "patient" or "mammal", is meant any subject, particularly a mammalian subject, e.g., a human patient, for whom diagnosis, prognosis, prevention, or therapy is desired.

"Maintaining a healthy brain" according to the invention can be understood as maintaining a normal neurological and brain function, wherein the normal cognitive function encompasses several domains, including memory, attention, concentration, alertness, mental flexibility and/or speed, learning, intelligence, language, problem solving capacity, consciousness, coping with psychological stress or tension, motivation, mobility, decision making and reaction time as well as emotions like anxiety and mood swings.

"Cognitive changes" relate to changes, for example, in memory, attention, concentration, alertness, mental flexibility and/or speed, learning, intelligence, language, problem solving capacity, consciousness, coping with psychological stress or tension, motivation, mobility, decision making and reaction time as well as emotions like anxiety and mood swings. These changes may be age or stress related. Furthermore, the changes may be mild, moderate or strong.

"A neurological condition" may refer to a state of an individual having one or more impaired neurological and/or cognitive functions. This state is milder than a disorder or disease, and may be caused, for example, by age or stress.

"Prevention of dementia, in particular Alzheimer disease, or improvement of the status of dementia, in particular Alzheimer disease" according to the invention can be understood as that the active ingredient has beneficial physiological effects to prevent or improve neurological function, cognitive functions, including memory, attention, concentration, alertness, mental flexibility and/or speed, learning, intelligence, language, problem solving capacity, consciousness, coping with psychological stress or tension, motivation, mobility, decision making and reaction time as well as emotions like anxiety and mood swings.

"Delaying onset of a dysfunction, condition, disorder or disease" can be understood as shifting the beginning of the dysfunction, condition, disorder or disease to a later time point.

"Controlling a dysfunction, condition, disorder or disease" means keeping or stabilizing a dysfunction, condition, disorder or disease at a level, which does not worsen dramatically.

"Dementia" means in this context a significant loss of intellectual abilities such as memory capacity, severe enough to interfere with social or occupational functioning.

"Alzheimer disease" means in this context a progressive neurologic disease of the brain that leads to the irreversible loss of neurons and dementia. The clinical hallmarks of Alzheimer's disease are progressive impairment in memory, judgment, decision making, orientation to physical surroundings, and language.

"Neurodegenerative diseases" include i.a. Alzheimer disease, Parkinson disease, Huntington disease, multiple sclerosis, amylotrophic lateral sclerosis (ALS), encephalitis or AIDS. [21]

Vicenin 2 is a flavonoid having a number of derivatizations. Vicenin 2 or its synonyms Apigenin-6,8-di-C-glycoside, 5,7,4'-Trihydroxyflavone-6,8-di-C-glucoside, 5,7-Dihydroxy-2-(4-hydroxyphenyl)-6,8-bis[(2S,3R,4R,5S,6R)-3,4,5-trihydroxy-6 (hydroxymethyl)oxan-2-yl]chromen-4-one with the CAS Registry Number 23666-13-9 is a flavonoid found in a number of plant species. Vicenin 2 can be found i.a. in Anethum, Perilla species, Urtica species, Passiflora species, Camelia species, Cayaponia species, Cydonia species, Colocasia species, Desmodium species, Hordeum species, Origanum species, Ocimum species, Jatropha species, Parkinsonia species, Peperomia species, Piheranthos species, Centaurea species, Indigo species, Bomba species, Lychnophera species, Asplenium species, Chinotto species, Citrus species, Viola species, Trigonella species, species belonging to the Lamiacea, Labiatae and e.g. Rosemary, Peppermint, Thyme, Basil, Sage, Oregano, Lavandula, Nipponanthemum, Abrus, Viola, Santalum, Oryza, Scleropyrum, Tulsi, Centaurea, Indigofera, Bombax, Glinus, Lychnophora and species belonging to Urticaceae, Rosales or Malpighiales.

The standard process to purify vicenin 2 out of plant material is based on different chromatographic methods known by person skilled in the art and consequently, vicenin 2 may be identified by spectroscopy. These processes can be reviewed in several publications. [13] So far the literature describes that vicenin 2 has an anti- cancer, anti-inflammatory and antinociceptive effect. [12, 13, 14]

"A plant extract comprising vicenin 2" is herein understood according to this invention to be an extract comprising vicenin 2 at a concentration which can be measured. The plant extract or preparation preferably comprises the active ingredient preferably at a concentration of at least 0.001%, 0.002%, 0.005%, 0.008%, 0.010% or at least 0.015% or from 0.02% to 0.3%, more preferably from 0.1% to 0.2% and preferably from 0.15% to 0.2%.

In some cases, the preparation may comprise the active ingredient at a concentration of at least 0.001%, more preferably, at a concentration of at least 0.002%, 0.005%, 0.008% or preferably at a concentration of at least 0.010%, 0.015% or 0.02%.

In some cases, the preparation may comprise the active ingredient at a concentration of at least 0.2%, more preferably, at a concentration of at least 0.5%, 1.5%, 3.0% or most preferably at a concentration of at least 2.0%, 3.0% or 5.0%.

There are several possibilities to investigate neurological effects, which are known to the skilled person. In vitro studies, like receptor binding or enzyme inhibition studies, are standard methods known to the person skilled in the art to identify activity and its mode of actions. This kind of test is shown in example 2. In addition it is possible to apply methods, which are known to the person skilled in the art and commonly used within the preclinical development phase of pharmaceutical agents in order to determine neurological activity when testing the acute neuroactive effects of substances on neuronal networks of murine cortex being grown on micro chips. By means of electrophysiological multi-channel recording, electrical potential are compared to benchmarks to identify potential effects as well as side effects. This kind of test is shown in example 1. [15, 16, 17]

Furthermore, in vivo animal and in vivo human studies are used to confirm the effect within the biological system. There are many well established animal tests to investigate neurological abilities like for example the forced swimming test. [18] Computer-based test for the assessment of working and short-term memory and selective attention are many times used to assess cognitive function and progression in cognitive disorders. [19] These kinds of studies are well known for the person skilled in the art.

In order to induce the beneficial neurological and brain health effects, the concentration of the active ingredient in the composition is 0.1 $\mu$g to 500 $\mu$g, preferably of 2.5$\mu$g to 50 $\mu$g and more preferably of 5 $\mu$g to 15$\mu$g or 12 $\mu$g to 30 $\mu$g, most preferably about 24 $\mu$g vicenin 2. The composition may be a plant preparation or plant extract, most preferably a liquid or powder extract obtained by extraction and comprising vicenin 2.

The composition according to the present invention may be comprised in a functional food product, dietary supplement or in a drug.

"A functional food product" according to this invention is understood to be a food, beverage or infant formular product, which offers, in addition, to nutritional value a health benefit, which supports and improves health and wellbeing or helps to reduce the risk to develop a disease.

"A dietary supplement product" according to this invention is a food product in form of a pill, tablet, capsule, pellet, globule, stick formulation, powder formulation, sachet formulation, powder or liquid form, which are meant to be taken by mouth, and contain substances like vitamins, minerals, foods, plant preparations, amino acids and are intended to supplement the usual intake of these substances via the normal diet.

"A medicament/ drug/ medicine" according to this invention is any substance with the potential to prevent or cure disease or enhance physical or mental welfare. If not stated otherwise the term "drug", "medicine", or "medicament" are used interchangeably herein and shall include but are not limited to all (A) articles, medicines and preparations for internal or external use, and any substance or mixture of substances intended to be used for diagnosis, cure, mitigation, treatment, or prevention of disease of either man or other animals; and (B) articles, medicines and preparations (other than food) intended to affect the structure or any function of the body of man or other animals; and (C) articles intended for use as a component of any article specified in clause (A) and (B). The term "drug", "medicine" or "medicament" shall include the complete formula of the preparation intended for use in either man or other animals containing one or more "agents", "ingredients", "compounds", "substances" or "(chemical) compositions" as and in some other context also other pharmaceutically inactive excipients as fillers, disintegrants, lubricants, glidants, binders or ensuring easy transport, disintegration, disaggregation, dissolution and biological availability of the "drug", "medicine", or "medicament" at an intended target location within the body of man or other animals, *e.g.,* at the skin, in the stomach or the intestine. The terms "agent", "compound" or "substance" are used interchangeably herein and shall include, in a more particular context, but are not limited to all pharmacologically active agents, *i.e.* agents that induce a desired biological or pharmacological effect or are investigated or tested for the capability of inducing such a possible pharmacological effect by the methods of the present invention.

"Pharmaceutically acceptable carrier" may include, but are not limited to aqueous nonaqueous base solutions, suspensions, emulsions, microemulsions, micellar solutions, gels and ointments. The pharmaceutically acceptable carrier may also contain ingredients that include, but are not limited to, saline and aqueous electrolyte solutions; ionic and nonionic osmotic agents such as sodium chloride, potassium chloride, glycerol, and dextrose; pH adjusters and buffers such as salts of hydroxide, hydronium, phosphate, citrate, acetate, borate, and tromethamine; antioxidants such as salts, acids and/or bases of bisulfite, sulfite, metabisulfite, thiosulfate, ascorbic acid, acetyl cysteine, cystein, glutathione, butylated hydroxyanisole, butylated hydroxytoluene, tocopherols, and ascorbyl palmitate; surfactants such as lecithin, phospholipids, including but not limited to phosphatidylcholine, phosphatidylethanolamine and phosphatidyl inositiol; poloxamers and poloxamines, polysorbates such as polysorbate 80, polysorbate 60, and polysorbate 20, polyethers such as polyethylene glycols and polypropylene glycols; polyvinyls such as polyvinyl alcohol and povidone; cellulose derivatives such as methylcellulose, hydroxypropyl cellulose, hydroxyethyl cellulose, carboxymethyl cellulose and hydroxypropyl methylcellulose and their salts; petroleum derivatives such as mineral oil and white petrolatum; fats such as lanolin, peanut oil, palm oil, soybean oil; mono-, di-, and triglycerides; polymers of acrylic acid such as carboxypolymethylene gel, and polysaccharides such as dextrans, and glycosaminoglycans such as sodium hyaluronate. Such pharmaceutically acceptable carriers may be preserved against bacterial contamination using well-known preservatives, these include, but are not limited to, benzalkonium chloride, ethylene diamine tetra-acetic acid and its salts, benzethonium chloride, chlorhexidine, chlorobutanol, methylparaben, thimerosal, and phenylethyl alcohol, or may be formulated as a non-preserved formulation for either single or multiple use.

As used herein, the terms "treat" or "treatment" refer to both therapeutic treatment and prophylactic or preventative measures, wherein the object is to prevent or slow down (lessen) an undesired physiological change or disorder, such as the development of a cognitive dysfunction, condition, disorder or disease. Beneficial or desired clinical results include, but are not limited to, alleviation of symptoms, diminishment of extent of disease, stabilization (*i.e.*, not worsening) state of disease, delay or slowing of disease progression, amelioration or palliation of the disease state, and remission (whether partial or total), whether detectable or undetectable. Those in need of treatment include those already with the condition or disorder as well as those prone to have the condition or disorder or those in which the manifestation of the condition or disorder is to be prevented.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" or "Pubmed" may be utilized, which is hosted by the National Center for Biotechnology Information and/or the National Library of Medicine at the National Institutes of Health. Further databases and web addresses, such as the virtual library "Martindale's center" are known to the person skilled in the art and can also be obtained using internet search engines.

The above disclosure generally describes the present invention. A more complete understanding can be obtained by reference to the following specific examples which are provided herein for purposes of illustration only.

## EXAMPLES

The examples which follow further illustrate the invention.

The practice of the present invention will employ, unless otherwise indicated, conventional techniques of plant biology, chemistry, biochemistry, physiology and pharmacology which are within the skill of the art.

**Example 1: Vicenin 2 and Perilla extract comprising Vicenin 2 and their neurological efficacy**

**Methods and Compounds**

Purpose of this example was the evaluation of the acute neuroactive effects of vicenin 2 (named AUC-V) and of Perilla extract (named AUC-P) comprising and standardized on Vicenin 2 on the neuronal activity of murine frontal cortex networks in vitro by means of electrophysiological multi-channel recordings. In this system, cells or tissues are grown directly on the chip surface and communicate via chemical and electrical signals. The MEA-neurochip allows the non-invasive, long-term, multisite recording of electrical signal patterns of primary neuronal networks. Said MEA-neurochip technology enables the characterization of network activity pattern on the single-cell action-potential level and on the level of complex neuronal networks as the basic functional units. This can also be used as a test system for investigating neuro-physiological properties of compounds. Despite the complexity, the neurophysiological action profiles of neuroactive compounds are sensitive and selective as well as robust and stable, allowing a precise pharmacological "fingerprinting" and the creation of a database of information on well-characterized neuroactive substances. The multiparametric description of the activity pattern changes caused by treatment of a brain-region-specific neuronal network is a sophisticated approach to quantify the complex effects of neuroactive agents, of unknown compounds, and of complex mixtures such as biological extracts. Correlation with well-known neuroactive substances and their specific pattern, available in specific databased, provides novel insights into the possible pharmacological and physiological mechanisms of modes of action of herbal preparations or isolated substances. [15, 16, 17]

In a first step the dose-effect curve of vicenin 2 (AUC-V) and the plant extract (AUC-P) were plotted by means of cumulatively increasing the substance concentration, so that the spectrum of activity of the substance is optimally covered with 9 concentrations. Concentration for Vicenin 2 from 100 fg/ml, 10 pg/ml, 100 pg/ml, 1 ng/ml, 100 ng/ml, 1 $\mu$g/ml, 10 $\mu$g/ml, 100 $\mu$g/ml and 300 $\mu$g/ml were used. Concentration for the plant extract from 10 ng/ml, 100 ng/ml, 1 $\mu$g/ml, 10 $\mu$g/ml, 30 $\mu$g/ml, 100 $\mu$g/ml, 200 $\mu$g/ml, 500 $\mu$g/ml, 1 mg/ml were used.

Subsequently, the measurements of the dose-effect curve of the test substances were repeated at least 10 times. The recorded electrical activity patterns were characterized by 200 features and their changes were statistically evaluated.

Afterwards, a further analysis was carried out through a pattern-recognition analysis and through comparison with NeuroProof database to determine relevant mechanisms involved in the frontal cortex activity pattern induced changes by each substance.

**Materials**

The chemicals 5-fluoro-2'-deoxyuridine + uridine (FDU), and poly-D-lysine were ordered from Sigma-Aldrich Chemical GmbH (Steinheim, Taufkirchen, Germany). DNase I (from bovine pancreas), and laminin were purchased from Roche (Mannheim, Germany), fetal bovine serum from Pan Biotech GmbH (Aidenbach, Germany), and accutase from PAA (Germany). Horse serum and Dulbecco's Modified Essential Medium (DMEM) were ordered from GIBCO BRL (Paisley, UK).

**Microelectrode Array Neurochips**

The microelectrode array neurochips (MEA neurochips) were provided by the Center for Network Neuroscience (CNNS) at the University of North Texas. These 5x5 $cm^2$ glass chips have a central recording matrix with 64 passive electrodes and indium tin oxide conductors. The hydrophobic insulation material surface was activated by a brief butane flame pulse through a stainless steel mask. Thus, cell attachment on a confined adhesive region (5 mm diameter centered on the electrode array) is ensured. The activated surface regions were coated with poly-D-lysine (25 $\mu$g/ml; 30-70kD) and laminin (16 $\mu$g/ml). Fabrication techniques and culture methods have been described previously.

**Cell Culture**

Frontal cortex tissue was harvested from embryonic day 15, or day 14 chr:NMRI mice. After ethyl ether anesthesia, mice were sacrificed by cervical dislocation according to the German Animal Protection Act §4. Neuronal tissue was cultured including the use of DNase I (8000 units/ml) and accutase (10 U/ml) for tissue dissociation. The tissue was dissociated enzymatically with accutase and mechanically with transfer pipettes. The cells were resuspended in DMEM 10/10 (10 % horse and 10 % fetal calf serum) at a density of 1.0 x $10^6$ cells/ml, and 400 $\mu$l were seeded onto MEA surfaces. Cultures were incubated at 37 °C in a 10% $CO_2$ atmosphere until ready for use, which usually is four weeks to three months after seeding. Culture media were replenished three times a week with DMEM containing 10% horse serum. Like in the tissue of origin, networks develop from a mixture of different types of postmitotic neurons and glial cells. The glial cells have important auxiliary functions for the metabolism and for supplying the neurons with ions and nutrients. The developing cocultures were treated with 5-fluoro-2'-deoxyuridine (25 $\mu$M) and uridine (63 $\mu$M) for 48 h to prevent further glial proliferation.

The cells growing directly on the neurochips emerge as natural neuronal networks. These are composed of a mixture of neurons and glial cells comparable to the tissue of origin, whereas in interaction with the neurons, the glial cells fulfill various metabolism and transport functions. The neurons were coupled electrically to the neurochip electrodes whereby the action potentials of the cells can be recorded and their amplitudes and the electrical activity pattern can be evaluated.

Activity starts after approximately three to four days in vitro in form of random spiking. Only after establishing a stable activity pattern after 4 weeks, the neuronal networks are employed in substance testing. For this study, cultures between 26 and 29 days in vitro were used.

### Multichannel Recording

For extracellular recording, MEA neurochips were placed into sterilized constant-bath recording chambers and maintained at 37 °C. Recordings were made in DMEM/10% horse serum. The pH was maintained at 7.4 with a continuous stream of filtered, humidified airflow with 10% $CO_2$. Sets of preamplifiers were positioned to either side of the recording chamber. Recording was performed with the multichannel acquisition processor system, a computer-controlled 64-channel amplifier system (Plexon, Inc., Dallas, TX, USA) providing programmable amplification, filtering, switching, and digital signal processing of microelectrode signals. The total system gain used was 10K with a simultaneous 40 kHz sampling rate. The signals routinely recorded by these neurochips are located in a range of 15-1800 $\mu$V.

The multichannel signal acquisition system delivered single neuron spike data. Spike identification and separation were accomplished with a template-matching algorithm in real time. This allowed the extracellular recording of action potentials from a maximum of 256 neurons simultaneously.

The action potentials, or "spikes", were recorded in spike trains and are clustered in so-called bursts. Bursts were quantitatively described via direct spike train analysis using the program NeuroEXplorer (Plexon Inc., Dallas, TX, USA) and in-house programs. Bursts were defined by the beginning and end of short spike invents. Maximum spike intervals defining the start of a burst were adjusted from 50 to 150 ms and maximum intervals to end a burst from 100 to 300 ms.

### Multiparametric Data Analysis

The high content analysis of the network activity patterns provided a multiparametric description characterizing the changes in four categories: general activity, burst structure, synchronicity and oscillatory behavior. The substance-specific activity changes were quantified by calculating for each stable activity phase after substance application a total of 200 activity-describing spike train parameters for these four categories below.

### Statistical Analysis

Results are expressed as series means $\pm$ SEM. The absolute parameters' distributions were tested for normality. The level of significance after compound application was assessed using Student's paired t-test. Significance between two substances or relative to a contained solvent (e.g. DMSO) was assessed using Student's unpaired t-test. $P < 0.05$ was considered statistically significant.

For direct comparability all parameters were normalized for each experiment and each experimental treatment with regard to the corresponding values of the reference activity (native or after receptor blockade if applicable set to 100%).

For each experiment, the changing spike rate as a function of the concentration was fitted to a one-sigmoidal or multiphasic-sigmoidal dose-response curve given by the equation:

$$y = y_{START} + \frac{y_{END} - y_{START}}{1 + 10^{[\log(EC_{50}) - \log(x)]*nH}}$$

determining the values of the effective concentration causing 10, 50, and 90% effect ($EC_{10}$, $EC_{50}$, and $EC_{90}$) and of the slope (Hill coefficient nH; describes the slope of the curve: a high value corresponds to steep decline which might correspond to functional neurotoxicity). In case of multiphasic response due to several mechanisms of action, the right term is repeatedly added for further phases.

## Pattern Recognition and Classification

To clarify the mode of action of the test substance on the activity of cortical networks these experiments were further analyzed using methods of pattern recognition. For each stable concentration activity phase the 200 spike train parameters were normalized by the native reference activity. These data records were computed for the test substances and the reference substances.

Using a feature selection algorithm, on the basis of the reference substances, the 40 most descriptive parameters for all 200 spike train parameters were selected. The rankings of activity features using various score methods based on classification experiments and comparing their total correct predictions were calculated. In this manner, the best results for a MDL (minimal description length) modified algorithm were obtained. A training data set with these 40 spike train parameters was established in the form of data records from the reference substances. An artificial neuronal network, multi layer feed forward network and back propagation algorithm without hidden units was then trained. The respective data records of the four substances were all subsequently classified. A classification against 105 substances in the database was carried out.

## Results:

Vicenin 2 showed an effect to induce a decrease of the cortical network activity with an $EC_{50}$ at 2 pg/ml and a maximum decrease to 80% of the native activity with no affects on the burst structure, however on the oscillatory behaviour of the network. This means that the undesired overload of the cortical networks can be diminished by vicenin 2. These results are shown in figure 1, which illustrates the concentration depending changes in the activity of the cortical network. It could be seen that vicenin 2 has an influence on the neuroactivity.

In addition, the extract comprising vicenin 2 has an effect to induce a decrease of the cortical network activity with an $EC_{50}$ at 90 ng/ml and 200 $\mu$g/ml and a maximum decrease to 16% of the native activity with no affects on the burst structure, however on the oscillatory behaviour of the network. These results are shown in figure 2, which illustrates the concentration depending changes in the activity of the cortical network. It could be seen that the extract comprising vicenin 2 has an influence on the neuroactivity.

In a second step, these activities were further evaluated by a classification against positive controls.

## Classifications for vicenin 2 and the Perilla extract

The classification was carried out against 105 substances using the electrophysiological multi-channel recording of vicenin 2 as well as the Perilla extract.

Table 1 shows the summaries of the 10 highest-ranking classifications.

| AUC-P, all | 122 | AUC-V, all | 143 |
|---|---|---|---|
| reference | # | reference | # |
| Muscimol | 25 | Olanzapine | 17 |
| Eserine | 24 | Eserine | 15 |
| DPDPE | 19 | Amisulpride | 15 |
| Acetaminophen | 18 | Enkephalin | 14 |
| Cortisol | 14 | Sodium dodecyl sulfate | 12 |
| Amitriptyline | 13 | Atropinemethylbromide | 12 |
| Amisulpride | 12 | Nicotine | 12 |
| Sodium propionate | 12 | Indatraline | 12 |
| Quetiapine | 11 | Modafinil | 12 |
| Carbenoxolone | 11 | DPDPE | 11 |

Table 1. Classification of vicenin 2 (AUC-V) and the extract (AUC-P) against the NeuroProof database. Shown is a ranking of classification results, which means that x-% of all data records of this substance were classified as the respective substance in the left column; DS corresponds to the relative overall ranking.

For example, it was possible to identify a sedative and anxiolytic activity of vicenin 2 as well as of the Perilla extract by the classification against Amisulpride, which is a known neuroleptica. It was also possible to identify a sedative and anxiolytic activity for Perilla extract by classification against Muscimol, which is a known GABA receptor agonist.

Surprisingly, it was also possible to identify an enkephalin-like activity for vicenin 2. Enkephalins are endogenous ligands that bind to the body's opioid receptors to modulate neuronal function, like learning or emotional behaviour, which can be altered by neurodegenerative disorders. In Alzheimer disease, elevated enkephalin levels may reflect compensatory or contribute to cognitive impairments.

Surprisingly, vicenin 2 a well as the extract comprising vicenin 2 showed also an electrophysiological multi-channel recording comparable with the pattern of the positive control eserine and nicotine. The extract shows also an electrophysiological multi-channel recording comparable with the pattern of the positive control eserine. Eserine and analoga are used in the treatment of Alzheimer disease as reversible acetylcholinesterase inhibitors to improve short term memory. [6, 7]

In addition, surprisingly vicenin 2 and the plant extract comprising the same demonstrated an electrophysiological multi-channel recording comparable with the pattern of the positive control of carboxolone. Carbenoxolone blocks gap junction hemichannel of activated microglia and reduces their glutamate release and consequently diminishes cell autonomous neuronal death in neurodegenerative diseases. Therefore, gap junction inhibitors are beneficial in treatment of neurodegenerative diseases. Carboxolone and analoga are used in the treatment of Alzheimer disease. [10]

Further, both the standardized extract as well as the isolated vicenin 2 demonstrated effects similar to amisulpride, which is a known neuroleptic agent. In addition, the extract showed an effect to anti-depressive amitripthyline and the active ingredient vicenin 2 to the anti-depressives and antipsychotics olanzapine and indatraline. Neuroleptika and antipsychotics are also used as companying therapy of Alzheimer to increase mood and motivation and to decrease anxiety and depression. This demonstrates further the effect of vicenin 2 and a composition comprising it in neurological and/or brain function and/or in maintaining and/or improving neurological and/or brain function and/or preventing, delaying onset, controlling and/or treating a neurological dysfunction, condition, disorder or disease.

Thus, example 1 surprisingly shows for the first time that vicenin 2 and an extract comprising the same (Perilla extract) can be used as a beneficial agent for neurological and brain functions or for the treatment of a cognitive disorder. Cognitive functions include, memory, attention (concentration), alertness, learning, intelligence, language, problem solving capacity, coping with psychological stress or tension, anxiety and mood alterations.

**Example 2: Perilla extract comprising and standardized on vicenin 2 has the ability to reduce TNF$\alpha$**

This study investigated the efficacy of Perilla extract comprising vicenin 2 to contribute to reduce TNF$\alpha$ levels. The study was designed as an ex vivo study, where human whole blood samples were treated with LPS, a bacterial blend, to stimulate inflammation which leads to an increase of the cytokine TNF$\alpha$. Furthermore, the effect to reduce TNF$\alpha$ by Perilla extract was measured in comparison to a negative and positive control. Perilla extract demonstrated a dose-depending effect to reduce TNF$\alpha$ (see figure 3).

Surprisingly, the extract as well as the isolated vicenin 2 demonstrated anti-inflammatory effects. Perilla extract demonstrated TNF$\alpha$ inhibiting efficacy within this ex vivo study showing a further beneficial effect in treatment if a cognitive disorder. In addition, TNF$\alpha$ has been recognized to be a gliatransmitter that regulates synaptic function in neural networks. Anti-TNF $\alpha$ treatment may thus lead to cognitive improvement. [8]

Even though anti-inflammatory agents have already been connected with Alzheimer disease, classical anti-inflammatory drugs, like NSAR, which act via the prostaglandin pathway most probably do not work in Alzheimer disease. Inflammation in the brain is mediated rather by activated microglial cells having an increased metabolic activity resulting in the production of proteins, which may contribute to the invasion of plaques. In addition, they produce inflammatory markers, like cytokines. Therefore, inhibition of cytokines, like tumor necrose factor-alpha (TNF$\alpha$), antioxidants and anti-inflammatory agents may also prevent the formation of amyloid beta, thus having a beneficial effect in Alzheimer disease [8].

Alzheimer is a multiplex disease and each person reacts differently on possible treatments. It is difficult to select the right treatment and therefore, ingredients which combine several mode of actions are preferred. However, before the present invention such agents having more than one effective mode of actions were lacking. Surprisingly, the present inventors showed that vicenin 2 as well as plant extracts comprising the same have more than one beneficial mode of actions for neurological and brain functions. This inventive agent may combine synergistically several mode of actions, i.e. at least blocking activity of gap junction hemichannel of activated microglia, inhibiting activity of tumor necrose factor $\alpha$ and inhibiting activity of acetylcholinesterase as well as having neuroleptic, anti-depressive effects and enkephaline like effects, and provides a new treatment possibility to maintain and improve neurological and/or brain function and to prevent and/or improve cognitive changes and to prevent and/or treat cognitive disorders, dementia including Alzheimer disease. The cognitive changes may be age or stress related and even mild.

Isolated vicenin 2 well as plant extracts comprising the same may combine synergistically several mode of actions being beneficial for maintenance and improvement of neurological and brain functions and for preventing and treating a cognitive disorder, dementia such as Alzheimer disease. The combination to offer several mode of actions within one agent is surprising and extremely beneficial as it contributes to a higher rate of success in the treatment of such a multi-complex and inter-individually disease like Alzheimer disease. The invention offers a highly effective beneficial agent and composition with a high compliance as individuals prefer to take one agent at once instead of many.

**Summary**

Summarizing, vicenin 2 as well as composition comprising the same demonstrated surprisingly beneficial neurological efficacy.

**References**

1. 2011 Alzheimer's Disease Facts and Figures, Alzheimer's Association, Alzheimer's & Dementia, Volume 7, Issue 2, 2011

2. Recommendations for the diagnosis and management of Alzheimer's disease and other disorders associated with dementia: EFNS guideline, Waldemar G. et al, European Journal of Neurology, 14, 1, 1-26, 2007

3. Recent developments in Alzheimer's disease therapeutics, Rafii M.S, Aisen, P.S. BMC Medicine, 7,7, 2009

4. Alzheimer disease: Time to improve, its diagnosis and treatment, Salloway S., Correia S, Cleveland Clinical Journal of Medicine, 76, 1, 2009

5. Alzheimer disease prevention: Focus on cardiovascular risk, not amyloid? Geldmacher D.S., Cleveland Clinical Journal of Medicine, 77, 10, 2010

6. The Experimental Alzheimer's Disease Drug Posiphen -Phenserine Lowers Amyloid-_ Peptide Levels in Cell Culture and Mice, Debomoy K. L. et al, The journal of pharmacology and experimental therapeutics, 320: 386-396, 2007

7. Phenserine, Klein J., Expert Opin Investig Drugs, 16 (7), 1087-97, 2007

8. Rapid cognitive improvement in Alzheimer's disease following perispinal etanercept administration, Tobinick E.L., Gross H., Journal of Neuroinflammation, 5:2, 2008

9. Investigational Medications for Treatment of Patients with Alzheimer Disease, Potter P.E., J Am Osteopath Assoc., 110 (9,8) 27-36, 2010

10. Blockade of Gap Junction Hemichannel Suppresses Disease Progression in Mouse Models of Amyotropic Lateral Sclerosis and Alzheimer's Disease, Takeuchi H. et al, PloS ONE, 6, 6, 2011

11. http://www.clinicaltrials.gov/ct2/results?term=alzheimer

12. Anti-cancer effects of novel flavonoid vicenin-2 as a single agent and in synergistic combination with docetaxel in prostate cancer. Nagaprashantha LD, et al., Biochem Pharmacol, 7, 2011

13. Vicenin-2, a potential anti-inflammatory constituent of Urtica circularis, Marrassini C, et al, J Nat Prod. 24;74(6):1503-7, 2011

14. Antinociceptive activity of ethanolic extract and isolated compounds of Urtica circularis. Gorzalczany S, et al, J Ethnopharmacol. :134(3):733-8, 2011

15. Nerve cell network in vitro: Applications to neurotoxicology, drug development, and biosensors Gross, G.W. et al, Cellular Engineering. 2: 138-147 (1997)

16. Quantification of acute neurotoxic effects of trimethyltin using neuronal networks cultured on microelectrode arrays, Gramowski, A. et al, NeuroToxicology. 21: 331-342 (2000)

17. Microelectrode arrays: A physiologically based neurotoxicity testing platform for the 21st century, Johnstone AFM et al, Neurotoxicology (2010), doi:10.1016/j.neuro.2010.04.001

18. Procognitive 5-HT6 antagonists in the rat forced swimming test: potential therapeutic utility in mood disorders associated with Alzheimer's disease, Hirano K et al, Life Sci. 10;84 (15-16):558-62, 2009

19. Computer-Based Assessment of Memory and Attention: Evaluation of the Memory and Attention Test (MAT), Adler G., Psychiatr Prax. 39(2):79-83, 201

20. Modulation of Brain Hemichannels and Gap Junction Channels by Pro-Inflammatory Agents and Their Possible Role in Neurodegeneration, Orellana J. et al., Antioxid Redox Signal 11 (2): (369-99), 2009

**Claims**

1. Vicenin 2 as an active ingredient for use in preventing, delaying onset, controlling and/or treating a neurological dysfunction, condition, disorder or disease, wherein the neurological dysfunction, condition, disorder or disease is an age or stress related neurological dysfunction, a cognitive disorder, depression or dementia, wherein dementia is preferably Alzheimer disease.

2. Non-therapeutic use of vicenin 2 as an active ingredient for maintaining and/or improving neurological and/or brain function,
   wherein the neurological and/or brain function is selected from the group consisting of memory, attention, concentration, alertness, mental flexibility and/or speed, learning, intelligence, language skills, problem solving capacity, consciousness, coping with psychological stress or tension, motivation, mobility, decision making capacity, reaction time and regulation of emotions.

3. The active ingredient for use according to claim 1 or the use according to claim 2, wherein the active ingredient is derived from a plant,
   preferably wherein the active ingredient is comprised in a plant preparation enriched for the active ingredient,
   and/or wherein the plant is selected of one or more plants from a group consisting of a Anethum, Perilla, Urtica, Passiflora, Camelia, Cayaponia, Colocasia, Cydonia, Desmodium, Hordeum, Origanum, Ocimum, Jatropha, Parkinsonia, Peperomia, Piheranthos, Centaurea, Indigo, Bomba, Lychnophera, Asplenium, Chinotto, Citrus, Viola, Trigonella, Rosemary, Peppermint, Thyme, Basil, Sage, Oregano, Lavandula, Nipponanthemum, Abrus, Viola, Santalum, Oryza, Scleropyrum, Tulsi, Centaurea, Indigofera, Bombax, Glinus, Lychnophora, other species belonging to the Lamiacea, Labiatae, and Urticaceae, Rosales or Malpighiales or a combination thereof,
   and/or wherein the active ingredient is derived from a plant preparation selected from the group consisting of a leaf preparation, a fruit preparation, a seed preparation, a stem preparation, a flower preparation, a bud preparation, a root preparation and a mixture of different parts of the plant.

4. The active ingredient for use according to any one of claims 1 or 3 or the use of claim 2 or 3, wherein the active ingredient is an isolated vicenin 2 obtained by isolation or chemical synthesis.

5. The active ingredient for use according to any one of claims 1, 3 or 4 or the use of any one of claims 2 to 4, wherein the active ingredient is comprised in a composition such as a food product, dietary supplement or medicament, preferably wherein the medicament comprises a pharmaceutically acceptable carrier.

6. The composition according to claim 5 for use according to claim 1 or the use of claim 5, wherein the concentration of the active ingredient for use is from 0.1 $\mu$g to 500 $\mu$g, preferably from 2.5 $\mu$g to 50 $\mu$g, preferably from 5 $\mu$g to 15$\mu$g, 12 $\mu$g to 30 $\mu$g or preferably about 24 $\mu$g,
   and/or which is substantially free of flavonoids of other plants, other plant extracts or other plant flavonoids.

7. The composition according to claim 5 or 6 for use according to claim 1 or the use of claim 5 or 6, comprising a further agent capable of maintaining and/or improving neurological and/or brain function and/or preventing, delaying onset, controlling and/or treating a neurological dysfunction, condition, disorder or disease as defined in any one of the preceding claims,
   preferably wherein the agent is a lipid, a lipid containing omega-3-fatty acid, physiological active fatty acid, an antioxidant, an anti-inflammatory agent, a bulking agent, an immune system modulatory agent or a vaccine, an antibody, a metal-protein interaction attenuation agent, a plant preparation, curcumin, coenzym Q10, L-carnitine, zinc, epigallocatechingallate, thymol, p-cymere, vinpocetine, hyperzine A, phosphatidylserine, a vitamin, alpha liponic acid, a TNF alpha inhibitor, a flavonoid, an anthocyanidin, a biflavonoid, a flavon, a flavonglycoside or a carboxylic acid,

preferably wherein the plant preparation is selected from one or more extracts from a group consisting of an extract of Ginkgo biloba, Hypericum perforatum, Hyperzia serrata, Galanthus nivalis, Salvia officinalis, Panex ginseng, Lippia citriodora, Melissa officinalis, Passiflora incarnate, Passiflora edulis, Bacopa monniera, Zingiber officinalis, Leucojum aestrum, Concolulus pluricaulis, Centella asiatica, Emblica officinalis, Coptidis Rhizoma, Salvia triloba, Piper nigrum, Trigonella foenum-graecum, Cimicifuga racemosa, Salvia miltiorrhiza, Rhodiola rosea, Habranthus jamesonii, Phycella herbertiana, Rhodophiala mendocina, Zephyranthes filifolia, Stephania pierrei, Kaempfera parviflora, Stephania venosa, Crocus sativus, Salvia species, Bacopa monnieri, Centella asiatica, Ptychopetalum olacoides, Withania somnifera, Coptis chinensis, Mangifera indica, Polygala caudata, Polygala tenuifolia, Halenia elliptica, Evolvilus alsinoides, Celastrus paniculatus, Clitoria ternatea, Curcuma longa, Acorus calamus, Terminalia chebula, Lycoris radiata, Magnolia officinalis, Biota orientalis, Codonopsis pilosula, Evodia rutaecarpa, Polygonum multiflorum, Aspalathus linearis, Cyclopia species, Adansonia digitata, Sclerocarya birrea, Mangifera indica, Actinidia chinensis and/or Matricaria recutita or combinations thereof,
and/or wherein the agent is an acetylcholinesterase inhibitor, a NMDA (N-methyl-D-aspartate) receptor inhibitor, a non steroidal anti-rheumatic agent, a neuroleptic agent, a tricyclic antidepressant, an anti-psychotic agent, a gab junction inhibitor, a non selective monoaminooxidase inhibitor, an ABCC1 Transporter, an ADAM 10 protein, methylthioninium chloride, an antibiotic agent, an antiviral agent, a gamma secretase inhibitor, a beta secretase inhibitor, an angiotensin receptor antagonist (AT1 antagonist), a cannabinoid, allopregnanolone or an insulin sensitizer.

8. The active ingredient for use as defined in any one of claims 1, 3 or 4, the composition of any one of claims 5 to 7 or the use of any one of the claims of 2 to 7 wherein the active ingredient, composition or preparation is packaged in a kit comprising instructions for administering said composition.

9. The active ingredient for use as defined in any one of claims 1 or 3 to 6, or the composition of claim 5 or 6 for use in preventing, delaying onset, controlling and/or treating a disorder associated with autonomous neuronal death by blocking the gap junction hemichannel of activated microglia and reducing their glutamate release,
preferably wherein the disorder is Alzheimer disease, Parkinson disease, Huntington disease, multiple sclerosis, amylotrophic lateral sclerosis (ALS), encephalitis or AIDS.

10. The active ingredient, the plant preparation or the composition for use according to claim 9, wherein the composition comprises or is derived of a plant preparation comprising the active ingredient, preferably wherein the plant preparation is derived from a plant and/or is plant preparation as defined in claim 3.

11. The active ingredient as defined in any one of claims 1, 3 or 4, or the composition of claim 5 or 6 having more than one effect selected from the group consisting of reversible acetylcholinesterase inhibition, blockade of gap junction hemichannels, neuroleptic effect, anti-depressive effect, enkephaline like effect and reduction in TNF alpha for use in preventing, delaying onset, controlling and/or treating neurological dysfunction, condition, disorder or disease, wherein the neurological dysfunction, condition, disorder or disease is an age or stress related neurological dysfunction, a cognitive disorder, depression or dementia.

12. The non-therapeutic use of any of claims 2 to 7 of vicenin 2 as an active ingredient having more than one effect selected from the group consisting of reversible acetylcholinesterase inhibition, blockade of gap junction hemichannels, neuroleptic effect, anti-depressive effect, enkephaline like effect and reduction in TNF alpha for maintaining and/or improving neurological and/or brain function.

**Patentansprüche**

1. Vicenin 2 als aktiver Inhaltstoff zur Verwendung in der Vorbeugung, Verzögerung des Beginns, Kontrolle und/oder Behandlung einer neurologischen Dysfunktion, eines Zustands, einer Störung oder Krankheit, wobei die neurologische Dysfunktion, Zustand, Störung oder Krankheit eine alters- oder stressbedingte neurologische Dysfunktion, eine kognitive Störung, Depression oder Demenz ist, wobei Demenz vorzugsweise Alzheimer-Krankheit ist.

2. Nicht-therapeutische Verwendung von Vicenin 2 als aktiver Inhaltstoff zur Aufrechterhaltung und/oder Verbesserung der neurologischen und/oder Gehirnfunktion,

EP 2 785 354 B1

wobei die neurologische und/oder Gehirnfunktion ausgewählt ist aus der Gruppe bestehend aus Gedächtnis, Aufmerksamkeit, Konzentration, Wachsamkeit, geistiger Flexibilität und/oder Geschwindigkeit, Lernen, Intelligenz, Sprachfähigkeit, Problemlösungskapazität, Bewusstsein, Bewältigung von psychischem Stress oder Spannung, Motivation, Mobilität, Entscheidungsfähigkeit, Reaktionszeit und Regulierung von Emotionen.

3. Aktiver Inhaltsstoff zur Verwendung nach Anspruch 1 oder Verwendung nach Anspruch 2, wobei der aktive Inhaltsstoff von einer Pflanze abgeleitet ist,
vorzugsweise wobei der aktive Inhaltstoff in einer Pflanzenzubereitung umfasst ist, die mit dem aktiven Inhaltstoff angereichert ist,
und/oder wobei die Pflanze ausgewählt ist aus einer oder mehreren Pflanzen aus einer Gruppe bestehend aus Anethum, Perilla, Urtica, Passiflora, Camelia, Cayaponia, Colocasia, Cydonia, Desmodium, Hordeum, Origanum, Ocimum, Jatropha, Parkinsonia, Peperomia, Piheranthos, Centaurea, Indigo, Bomba, Lychnophera, Asplenium, Chinotto, Zitrusfrüchte, Viola, Trigonella, Rosmarin, Pfefferminze, Thymian, Basilikum, Salbei, Oregano, Lavendel, Nipponanthemum, Abrus, Viola, Santalum, Oryza, Scleropyrum, Tulsi, Centaurea, Indigofera, Bombax, Glinus, Lychnophora, andere Arten, die zu den Lamiacea, Labiatae und Urticaceae gehören, Rosales oder Malpighiales oder eine Kombination davon,
und/oder wobei der aktive Inhaltstoff von einer Pflanzenzubereitung abgeleitet ist, die aus der Gruppe ausgewählt ist bestehend aus Blattzubereitung, Fruchtzubereitung, Samenzubereitung, Stammzubereitung, Blütenzubereitung, Knospenzubereitung, Wurzelzubereitung und einer Mischung verschiedener Pflanzenteile.

4. Aktiver Inhaltstoff zur Verwendung nach einem der Ansprüche 1 oder 3 oder die Verwendung nach Anspruch 2 oder 3, wobei der aktive Inhaltstoff ein isoliertes Vicenin 2, das durch Isolierung oder chemische Synthese erhalten wurde, ist.

5. Aktiver Inhaltstoff nach einem der Ansprüche 1, 3 oder 4 oder die Verwendung nach einem der Ansprüche 2 bis 4, wobei der aktive Inhaltstoff in einer Zusammensetzung, wie einem Nahrungsmittel, einem Nahrungsergänzungsmittel oder einem Medikament, umfasst ist, vorzugsweise wobei das Medikament einen pharmazeutisch annehmbaren Träger umfasst.

6. Zusammensetzung nach Anspruch 5 zur Verwendung nach Anspruch 1 oder Verwendung nach Anspruch 5, wobei die Konzentration des aktiven Inhaltstoffs von 0,1 $\mu$g bis 500 $\mu$g, vorzugsweise von 2,5 $\mu$g bis 50 $\mu$g, vorzugsweise von 5 $\mu$g bis 15$\mu$g, 12 $\mu$g bis 30 $\mu$g oder vorzugsweise etwa 24 $\mu$g beträgt,
und/oder die im wesentlichen frei von Flavonoiden anderer Pflanzen, anderen Pflanzenextrakten oder anderen Pflanzenflavonoiden ist.

7. Zusammensetzung nach Anspruch 5 oder 6 zur Verwendung nach Anspruch 1 oder Verwendung nach Anspruch 5 oder 6, umfassend ein weiteres Mittel, das in der Lage ist, eine neurologische und/oder Gehirnfunktion aufrechtzuerhalten und/oder zu verbessern und/oder eine neurologische Dysfunktion, Zustand, Störung oder Krankheit, wie in einem der vorhergehenden Ansprüche definiert, zu verhindern, den Beginn zu verzögern, zu kontrollieren und/oder zu behandeln,
vorzugsweise wobei das Mittel ein Lipid, ein Omega-3-Fettsäure enthaltendes Lipid, physiologisch aktive Fettsäure, ein Antioxidationsmittel, ein entzündungshemmendes Mittel, ein Füllstoff, ein immunsystemmodulierendes Mittel oder ein Impfstoff, ein Antikörper, ein Mittel zur Abschwächung der Metall-Protein-Interaktion ist, ein Pflanzenpräparat, Curcumin, Coenzym Q10, L-Carnitin, Zink, Epigallocatechingallat, Thymol, p-Cymere, Vinpocetin, Hyperzin A, Phosphatidylserin, ein Vitamin, Alpha-Liponsäure, ein TNF-Alpha-Inhibitor, ein Flavonoid, ein Anthocyanidin, ein Biflavonoid, ein Flavon, ein Flavonglycosid oder eine Carbonsäure ist,
vorzugsweise, wobei die Pflanzenzubereitung ausgewählt ist aus einem oder mehreren Extrakten aus einer Gruppe bestehend aus einem Extrakt von Ginkgo biloba, Hypericum perforatum, Hyperzia serrata, Galanthus nivalis, Salvia officinalis, Panex ginseng, Lippia citriodora, Melissa officinalis, Passiflora incarnate, Passiflora edulis, Bacopa monniera, Zingiber officinalis, Leucojum aestrum, Concolulus pluricaulis, Centella asiatica, Emblica officinalis, Coptidis Rhizoma, Salvia triloba, Piper nigrum, Trigonella foenum-graecum, Cimicifuga racemosa, Salvia miltiorrhiza, Rhodiola rosea, Habranthus jamesonii, Phycella herbertiana, Rhodophiala mendocina, Zephyranthes filifolia, Stephania pierrei, Kaempfera parviflora, Stephania venosa, Crocus sativus, Salvia-Arten, Bacopa monnieri, Centella asiatica, Ptychopetalum olacoides, Withania somnifera, Coptis chinensis, Mangifera indica, Polygala caudata, Polygala tenuifolia, Halenia elliptica, Evolvilus alsinoides, Celastrus paniculatus, Clitoria ternatea, Curcuma longa, Acorus calamus, Terminalia chebula, Lycoris radiata, Magnolia officinalis, Biota orientalis, Codonopsis pilosula, Evodia rutaecarpa, Polygonum multiflorum, Aspalathus linearis, Cyclopia-Arten, Adansonia digitata, Sclerocarya birrea, Mangifera indica, Actinidia chinensis und/oder Matricaria recutita oder Kombinationen davon,

und/oder wobei das Mittel Acetylcholinesterase-Inhibitor, NMDA-(N-Methyl-D-Aspartat)-Rezeptor-Inhibitor, nicht steroidales Antirheumatikum, Neuroleptikum, trizyklisches Antidepressivum, Antipsychotikum, Gab Junction-Inhibitor, ein nichtselektiven Monoaminooxidase-Inhibitor, ABCCI-Transporter, ADAM 10-Protein, Methylthioniniumchlorid, Antibiotikum, antivirales Mittel, Gamma-Sekretase-Inhibitor, Beta-Sekretase-Inhibitor, Angiotensin-Rezeptor-Antagonisten (AT1-Antagonist), Cannabinoid, Allopregnanolon oder Insulin-Sensibilisator ist.

8. Aktiver Inhaltstoff zur Verwendung wie in einem der Ansprüche 1, 3 oder 4 definiert, Zusammensetzung nach einem der Ansprüche 5 bis 7 oder die Verwendung nach einem der Ansprüche 2 bis 7, wobei der aktive Inhaltstoff, Zusammensetzung oder Zubereitung in einem Kit, umfassend einer Anleitung zur Verabreichung dieser Zusammensetzung, verpackt ist.

9. Aktiver Inhaltstoff zur Verwendung wie in einem der Ansprüche 1 oder 3 bis 6 definiert, oder die Zusammensetzung nach Anspruch 5 oder 6 zur Verwendung bei der Vorbeugung, Verzögerung des Beginns, Kontrolle und/oder Behandlung einer Erkrankung, die mit dem autonomen neuronalen Tod durch Blockierung des Gap Junction-Halbkanals aktivierter Mikroglia und Verringerung ihrer Glutamatfreisetzung verbunden ist,
vorzugsweise wobei die Störung Alzheimer-Krankheit, Parkinson-Krankheit, Huntington-Krankheit, Multiple Sklerose, amylotrophe Lateralsklerose (ALS), Enzephalitis oder AIDS ist.

10. Aktiver Inhaltstoff, Pflanzenzubereitung oder Zusammensetzung zur Verwendung nach Anspruch 9, wobei die Zusammensetzung umfasst oder abgeleitet ist von einer Pflanzenzubereitung umfassend den aktiven Inhaltstoff,
vorzugsweise wobei die Pflanzenzubereitung von einer Pflanze abgeleitet ist und/oder eine Pflanzenzubereitung ist, wie in Anspruch 3 definiert ist.

11. Aktiver Inhaltstoff wie in einem der Ansprüche 1, 3 oder 4 definiert, oder die Zusammensetzung nach Anspruch 5 oder 6 mit mehr als einer Wirkung, ausgewählt aus der Gruppe bestehend aus reversibler Acetylcholinesterase-Inhibition, Blockade von Gap Junction-Halbkanälen, neuroleptische Wirkung, antidepressive Wirkung, enkephalinartige Wirkung und Verminderung von TNF-alpha zur Verwendung zur Vorbeugung, Verzögerung des Beginns, Kontrolle und/oder Behandlung von neurologische Dysfunktion, Zustand, Störung oder Krankheit, wobei die neurologische Dysfunktion, Zustand, Störung oder Krankheit ein alters- oder stressbedingte neuronale Dysfunktion, eine kognitive Störung, Depression oder Demenz ist.

12. Nicht-therapeutische Verwendung nach einem der Ansprüche 2 bis 7 von Vicenin 2 als ein aktiver Inhaltstoff mit mehr als einer Wirkung, ausgewählt aus der Gruppe bestehend aus reversibler Acetylcholinesterase-Inhibition, Blockade von Gap Junction-Halbkanälen, neuroleptische Wirkung, antidepressive Wirkung, enkephalinartige Wirkung und Verminderung von TNF-alpha zur Aufrechterhaltung und/oder Verbesserung der neurologischen und/oder Gehirnfunktion.

## Revendications

1. Vicénine 2 en tant qu'ingrédient actif à utiliser pour prévenir, retarder l'apparition, commander et/ou traiter un dysfonctionnement, un état, un trouble ou une maladie neurologique, où le dysfonctionnement, l'état, le trouble ou la maladie neurologique est un dysfonctionnement neurologique lié à l'âge ou au stress, un trouble cognitif, une dépression ou une démence, la démence étant de préférence la maladie d'Alzheimer.

2. Utilisation non thérapeutique de vicénine 2 en tant qu'ingrédient actif pour maintenir et/ou améliorer la fonction neurologique et/ou cérébrale,
où la fonction neurologique et/ou cérébrale est choisie dans le groupe comprenant la mémoire, l'attention, la concentration, la vigilance, la souplesse et/ou la vitesse mentale(s), l'apprentissage, l'intelligence, les compétences linguistiques, la capacité à résoudre un problème, la conscience, la gestion du stress ou de la tension psychologique, la motivation, la mobilité, la capacité de décision, le temps de réaction et la régulation des émotions.

3. Ingrédient actif à utiliser selon la revendication 1 ou utilisation selon la revendication 2, où l'ingrédient actif est dérivé d'une plante,
de préférence où l'ingrédient actif est compris dans une préparation végétale enrichie pour l'ingrédient actif,
et/ou où la plante est choisie parmi une ou plusieurs plantes d'un groupe comprenant anéthum, perilla, urtica, passiflora, camélia, cayaponia, colocasia, cydonia, desmodium, hordeum, origanum, ocimum, jatropha, parkinsonia, peperomia, piheranthos, Centaurea, Indigo, Bomba, Lychnophera, Asplenium, Chinotto, Citrus, Viola, Trigonella, Romarin, Menthe poivrée, Thym, Basilic, Sauge, Origan, Lavandula, Nipponanthemum, Abrus, Viola, Santalum, Oryza, Scleropyrum, Tulsi, Centaurea, Indigofera, Bombax, Glinus, Lychnophora, d'autres espèces appartenant aux Lamiacea, Labiatae et Urticaceae, Rosales ou Malpighiales, ou une combinaison de celles-ci, et/ou où l'ingrédient actif est dérivé d'une préparation végétale choisie dans le groupe consistant en une préparation de feuilles, une préparation de fruits, une préparation de graines, une préparation de tiges, une préparation de fleurs, une préparation de bourgeons, une préparation de racines et un mélange de différentes parties de la plante.

4. Ingrédient actif à utiliser selon la revendication 1 ou utilisation selon la revendication 3, où l'ingrédient actif est une vicénine 2 isolée obtenue par isolement ou synthèse chimique.

5. Ingrédient actif à utiliser selon la revendication 1, 3 ou 4 ou utilisation selon l'une quelconque des revendications 2 à 4, où l'ingrédient actif est compris dans une composition telle qu'un produit alimentaire, un complément alimentaire ou un médicament, de préférence où le médicament comprend un support pharmaceutiquement acceptable.

6. Composition selon la revendication 5 pour une utilisation selon la revendication 1 ou utilisation selon la revendication 5, où la concentration de l'ingrédient actif à utiliser est de 0,1 $\mu$g à 500 $\mu$g, de préférence de 2,5 $\mu$g à 50 $\mu$g, de préférence de 5 $\mu$g à 15 $\mu$g, de 12 $\mu$g à 30 $\mu$g ou de préférence d'environ 24 $\mu$g, et/ou qui est sensiblement exempte de flavonoïdes d'autres plantes, d'autres extraits de plante ou d'autres flavonoïdes de plante.

7. Composition selon la revendication 5 ou 6 à utiliser selon la revendication 1 ou utilisation selon la revendication 5 ou 6, comprenant un agent supplémentaire capable de maintenir et/ou d'améliorer la fonction neurologique et/ou cérébrale et/ou de prévenir, retarder l'apparition, commander et/ou traiter un dysfonctionnement , un état, un trouble ou une maladie neurologique tels que définis dans l'une quelconque des revendications précédentes, de préférence où l'agent est un lipide, un lipide contenant un acide gras oméga-3, un acide gras physiologiquement actif, un antioxydant, un agent anti-inflammatoire, un agent gonflant, un agent modulateur du système immunitaire ou un vaccin, un anticorps, un agent d'atténuation d'interaction métal-protéine, une préparation végétale, la curcumine, la coenzyme Q10, la L-carnitine, le zinc, l'épigallocatéchingallate, le thymol, le p-cymène, la vinpocétine, l'hyperzine A, la phosphatidylsérine, une vitamine, l'acide alpha-liponique, un inhibiteur de TNF alpha, un flavonoïde, une anthocyanidine, un biflavonoïde, une flavone, un flavonglycoside ou un acide carboxylique, de préférence où la préparation végétale est choisie parmi un ou plusieurs extraits d'un groupe constitué d'un extrait des espèces Ginkgo biloba, Hypericum perforatum, Hyperzia serrata, Galanthus nivalis, Salvia officinalis, Panex ginseng, Lippia citriodora, Melissa officinalis, Passiflora incarnate, Passiflora edulis, Bacopa monniera, Zingiber officinalis, Leucojum aestrum, Concolulus pluricaulis, Centella asiatica, Emblica officinalis, Coptidis Rhizoma, Salvia triloba, Piper nigrum, Trigonella foenum-graecum, Cimicifuga racemosa, Salvia miltiorrhiza, Rhodiola rosea, Habranthus jamesonii, Phycella herbertiana, Rhodophiala mendocina, Zephyranthes filifolia, Stephania pierrei, Kaempfera parviflora, Stephania venosa, Crocus sativus, Salvia species, Bacopa monnieri, Centella asiatica, Ptychopetalum olacoides, Withania somnifera, Coptis chinensis, Mangifera indica, Polygala caudata, Polygala tenuifolia, Halenia elliptica, Evolvilus alsinoides, Celastrus paniculatus, Clitoria ternatea, Curcuma longa, Acorus calamus, Terminalia chebula, Lycoris radiata, Magnolia officinalis, Biota orientalis, Codonopsis pilosula, Evodia rutaecarpa, Polygonum multiflorum, Aspalathus linearis, Cyclopia, d'Adansonia digitata, de Sclerocarya birrea, de Mangifera indica, d'Actinidia chinensis et/ou de Matricaria recutita, ou des combinaisons de ceux-ci, et/ou où l'agent est un inhibiteur d'acétylcholinestérase, un inhibiteur de récepteur NMDA (N-méthyl-D-aspartate), un agent antirhumatismal non stéroïdien, un agent neuroleptique, un antidépresseur tricyclique, un agent antipsychotique, un inhibiteur de jonction gab, un inhibiteur de monoaminooxydase non sélectif, un transporteur ABCC1, une protéine ADAM 10, du chlorure de méthylthioninium, un agent antibiotique, un agent antiviral, un inhibiteur de gamma sécrétase, un inhibiteur de bêta sécrétase, un antagoniste de récepteur d'angiotensine (antagoniste AT1), un cannabinoïde, l'alloprégnanolone ou un sensibilisateur à l'insuline.

8. Ingrédient actif à utiliser tel que défini dans l'une quelconque des revendications 1, 3 ou 4, composition selon l'une quelconque des revendications 5 à 7 ou utilisation selon l'une quelconque des revendications 2 à 7, où l'ingrédient actif, la composition ou la préparation est conditionné(e) dans un kit comprenant des instructions pour une administration de ladite composition.

9. Ingrédient actif à utiliser tel que défini dans l'une quelconque des revendications 1 ou 3 à 6, ou composition selon la revendication 5 ou 6 à utiliser pour prévenir, retarder l'apparition, commander et/ou traiter un trouble associé à la mort neuronale autonome en bloquant l'hémicanal de jonction lacunaire de microglies activées et en réduisant leur libération de glutamate,

de préférence où le trouble est la maladie d'Alzheimer, la maladie de Parkinson, la maladie de Huntington, la sclérose en plaque, la sclérose latérale amylotrophique (SLA), l'encéphalite ou le SIDA.

10. Ingrédient actif, préparation végétale ou composition à utiliser selon la revendication 9, où la composition comprend ou est dérivée d'une préparation végétale comprenant l'ingrédient actif, de préférence où la préparation végétale est dérivée d'une plante et/ou est une préparation de plantes telle que définie dans la revendication 3.

11. Ingrédient actif tel que défini dans l'une quelconque des revendications 1, 3 ou 4, ou composition selon la revendication 5 ou 6 ayant plus d'un effet choisi dans le groupe constitué par une inhibition réversible de l'acétylcholinestérase, un blocage d'hémicanaux à jonction lacunaire, un effet neuroleptique, un effet anti-dépressif, un effet semblable à l'enképhaline et une réduction de TNF alpha pour la prévention, le retardement de l'apparition, la commande et/ou le traitement d'un dysfonctionnement, d'un état, d'un trouble ou d'une maladie neurologique, où le dysfonctionnement, l'état, le trouble ou la maladie neurologique est un dysfonctionnement neurologique lié à l'âge ou au stress, un trouble cognitif, une dépression ou une démence.

12. Utilisation non thérapeutique de l'une quelconque des revendications 2 à 7 de vicénine 2 en tant qu'ingrédient actif ayant plus d'un effet choisi dans le groupe consistant en une inhibition d'acétylcholinestérase réversible, un blocage d'hémicanaux à jonction lacunaire, un effet neuroleptique, un effet anti-dépressif, un effet analogue à l'enképhaline et une réduction de TNF alpha pour maintenir et/ou améliorer une fonctions neurologique et/ou cérébrale.

**Fig. 1**

Fig. 2

**TNF-alpha**

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SENA et al.** *Experimental Biology and Medicine,* 2009, vol. 234, 967-975
- Industrial Scale Natural Products Extraction. Wiley-VCH, 2011
- Alzheimer's Disease Facts and Figures, Alzheimer's Association. *Alzheimer's & Dementia,* 2011, vol. 7 (2
- **WALDEMAR G. et al.** Recommendations for the diagnosis and management of Alzheimer's disease and other disorders associated with dementia: EFNS guideline. *European Journal of Neurology,* 2007, vol. 14 (1), 1-26
- Recent developments. **RAFII M.S ; AISEN, P.S.** Alzheimer's disease therapeutics. BMC Medicine, 2009, vol. 7, 7
- **SALLOWAY S. ; CORREIA S.** Alzheimer disease: Time to improve, its diagnosis and treatment. *Cleveland Clinical Journal of Medicine,* 2009, vol. 76 (1
- Alzheimer disease prevention: Focus on cardiovascular risk, not amyloid? Geldmacher D.S. *Cleveland Clinical Journal of Medicine,* 2010, vol. 77, 10
- **DEBOMOY K. L. et al.** The Experimental Alzheimer's Disease Drug Posiphen -Phenserine Lowers Amyloid-_ Peptide Levels in Cell Culture and Mice. *The journal of pharmacology and experimental therapeutics,* 2007, vol. 320, 386-396
- **PHENSERINE, KLEIN J.** *Expert Opin Investig Drugs,* 2007, vol. 16 (7), 1087-97
- **TOBINICK E.L. ; GROSS H.** Rapid cognitive improvement in Alzheimer's disease following perispinal etanercept administration. *Journal of Neuroinflammation,* 2008, vol. 5, 2
- **POTTER P.E.** Investigational Medications for Treatment of Patients with Alzheimer Disease. *J Am Osteopath Assoc.,* 2010, vol. 110 (9,8), 27-36

- **TAKEUCHI H. et al.** Blockade of Gap Junction Hemichannel Suppresses Disease Progression in Mouse Models of Amyotropic Lateral Sclerosis and Alzheimer's Disease. *PloS ONE,* 2011, vol. 6, 6
- **NAGAPRASHANTHA LD et al.** Anti-cancer effects of novel flavonoid vicenin-2 as a single agent and in synergistic combination with docetaxel in prostate cancer. *Biochem Pharmacol,* 2011, vol. 7
- Vicenin-2, a potential anti-inflammatory constituent of Urtica circularis, Marrassini C. *J Nat Prod.,* 2011, vol. 74 (6), 1503-7
- **GORZALCZANY S et al.** Antinociceptive activity of ethanolic extract and isolated compounds of Urtica circularis. *J Ethnopharmacol.,* 2011, vol. 134 (3), 733-8
- **GROSS, G.W. et al.** Nerve cell network in vitro: Applications to neurotoxicology, drug development, and biosensors. *Cellular Engineering,* 1997, vol. 2, 138-147
- **GRAMOWSKI, A. et al.** Quantification of acute neurotoxic effects of trimethyltin using neuronal networks cultured on microelectrode arrays. *NeuroToxicology,* 2000, vol. 21, 331-342
- **JOHNSTONE AFM et al.** Microelectrode arrays: A physiologically based neurotoxicity testing platform for the 21st century. *Neurotoxicology,* 2010
- **HIRANO K et al.** Procognitive 5-HT6 antagonists in the rat forced swimming test: potential therapeutic utility in mood disorders associated with Alzheimer's disease. *Life Sci.,* 2009, vol. 84, 558-62
- **ADLER G.** Computer-Based Assessment of Memory and Attention: Evaluation of the Memory and Attention Test (MAT). *Psychiatr Prax,* vol. 39 (2), 79-83
- **ORELLANA J. et al.** Modulation of Brain Hemichannels and Gap Junction Channels by Pro-Inflammatory Agents and Their Possible Role in Neurodegeneration. *Antioxid Redox Signal,* 2009, vol. 11 (2), 369-99